# EUROPEAN PATENT APPLICATION

(11) **EP 4 821 524 A1**
(43) Date of publication of application: **14.10.2026**
(21) Application number: 25170146.2
(22) Date of filing: 11.04.2025
(51) Int. Cl.: A61M 5/32, A61M 5/34, A61M 37/00, A61M 39/22, A61M 39/10

(54) **A SYRINGE COUPLING AND MICRONEEDLE BASED DELIVERY DEVICE FOR USE WITH SAME**

(71) Applicant: Latch Medical Limited, 4 Dublin (IE)
(72) Inventor: BERTOLLO, Nicky, Dublin, 24 (IE)
(74) Representative: FRKelly

(57) **Abstract**

16. The invention provides a syringe coupling which enables a conventional syringe to be used with a microneedle based drug delivery device, the syringe coupling being sealingly connectable to the barrel of the syringe and having a head sealingly connectable to the microneedle based drug delivery device, the syringe coupling further including a seal that is automatically ruptured as the microneedle based drug delivery device is deployed onto target tissue.

## Description

### Field of the invention

This invention relates to a syringe coupling to allow a conventional syringe containing a medicament, for example a vaccine or other injectable drug, to be hermetically sealed following filling with the medicament, and to be selective fluidically connected to a microneedle based delivery system through which the medicament may be delivered from the syringe into target tissue via one or more microneedles.

### Background of the invention

Microneedles are gaining increased use in various medical applications in view of the many documented benefits for both patients and medical professionals, for example reduced tissue trauma, reduced risk of infection, and minimising the need for medical professional intervention when using such microneedle based devices in applications such as drug delivery or the like. Such microneedles allow for extremely accurate, and shallow, deployment into tissue, which can be particularly beneficial in applications such as drug delivery to sensitive or delicate tissue such as skin, allowing reliable and repeatable delivery of medicaments which may therefore reduce overheads on medical professionals.

Intradermal delivery of drugs and vaccines offers significant advantages over conventional intramuscular and oral delivery routes for vaccines and drugs. Systemic uptake of therapeutics via the dermal blood capillaries and lymphatic system offers a host of benefits, including avoiding the deleterious effects of first-pass metabolism, rapid drug onset and improved bioavailability of APIs, such as biologics, that are not readily absorbed across the mucosal layers of the gastrointestinal tract. Additionally, the dermal layer of skin is replete with antigen presenting cells and therefore represents an optimal location for the delivery of vaccines in order to elicit an enhanced immune response, and in some cases, using lower dosages relative to standard intramuscular administration.

International patent applications WO2018/069543 and WO2019/201903 provide detailed disclosures of the configuration and operation of devices employing opposing microneedle arrays for application to a tissue substrate to facilitate drug delivery directly through the microneedles provided, which also provide a temporary anchoring function to retain the device in contact with the target tissue during drug delivery. The disclosures of WO2018/069543 and WO2019/201903 are incorporated herein in their entirety.

Using the above disclosed devices allows opposed sets of microneedles to be quickly and easily placed in contact with the skin or other tissue, which microneedles are then displaced into a deployed state or condition in which the microneedles are inserted into the skin, through relative displacement of the sets of microneedles. This may be achieved my means of relative displacement between a pair of device sections carrying the microneedles, in a direction generally parallel to the skin or tissue surface. This transverse or lateral displacement acts to insert the angled microneedles into the skin, with the opposed sets of microneedles overlapping and acting to grip the intervening skin and thus retain or anchor the microneedles in place in order to ensure stability during use and therefore accurate and consistent injection of the liquid medicament. This anchoring action is also described in detail in earlier International applications WO2018/069543, WO2022/238411, and WO2024/052432, and no further explanation is therefore necessary in relation to this particular aspect of the invention disclosed herein.

As also disclosed in detail in WO2022/238411, one particular drug delivery application utilises a microneedle based device to which an otherwise conventional syringe may be connected, in order to allow a medicament contained in the syringe to be delivered through the microneedles of the device into the skin or other tissue when the syringe plunger is depressed in conventional fashion. The device includes a manifold via which fluid communication with the syringe may be established, the manifold defining one or more fluid conduits extending through the manifold to the respective microneedles. In this way a fluid path is established from the syringe, through the fluid manifold and to the microneedles. These operations can also be automated in a microneedle based delivery device, as is also described in WO2024/052432.

There is nevertheless a need for an improved interface between the syringe and the microneedle based delivery devices described above, which is simple to use, is reliable, and which can provide a suitable seal to the free end of the filled syringe until the medicament contained therein is to be administered.

It is therefore an object of the present invention to address the above mentioned problem.

### Summary of the invention

According to a first aspect of the present invention there is provided a syringe coupling comprising a hub having a first end sealingly connectable to a barrel of a syringe and an opposed second end between which is defined a fluid flow lumen; a head provided at the second end of the hub and comprising an lower face defining at least one opening therein, the head further comprising at least one passage communicating between the lumen and the at least one opening; and a seal located about the at least one opening and displaceable from a first state occluding the opening to a second state exposing the opening.

Preferably, the seal comprises a deformable material.

Preferably, the seal comprises a plug at least partially disposed in the at least one opening.

Preferably, the seal comprises a rupturable membrane extending over at least the at least one opening.

Preferably, the syringe coupling further comprises at least one gasket on the lower face and surrounding the at least one opening.

Preferably, the at least one gasket comprises a compressible material.

Preferably, the syringe coupling comprises a torque plate operable to prevent relative rotation of the syringe coupling when captured with a microneedle based delivery device.

Preferably, the torque plate extends transversely to a longitudinal axis of the hub.

Preferably, the torque plate is rectangular.

Preferably, the torque plate is located intermediate the hub and the head.

Preferably, the hub comprises a thread for engaging with a corresponding thread on the syringe.

Preferably, the lower face defines a pair of openings therein, the head further comprising a corresponding pair of passages communicating between the lumen and the pair of openings.

According to a second aspect of the present invention there is provided a microneedle based drug delivery device comprising a body having a first section and a second section displaceable relative to one another; at least one first microneedle provided on the first section and at least one second microneedle provided on the second section, at least one of the first or second microneedles being hollow such as to facilitate drug delivery therethrough, the first and second sections being displaceable relative to one another in order to transition the first and second microneedles between a disengaged state and an engaged state; a delivery manifold in fluid communication with the at least one hollow microneedle and defining a sealing face against which the lower face of the head of the syringe coupling of the first aspect of the invention is sealable engagable; and at least one breaching element displaceable in response to relative displacement of the first and second sections such as to effect displacement of the seal from the first state into the second state.

Preferably, the syringe coupling is captured between the first and second sections at least when the microneedles are in the engaged state.

Preferably, the lower face is urged against the seat when the microneedles are in the engaged state such as to establish a fluid tight seal between the lower face and the seat.

Preferably, the microneedle based delivery system comprises the syringe coupling of the first embodiment and a syringe fluidically connected to the syringe coupling.

### Brief description of the drawings

The present invention will now be described with reference to the accompanying drawings, in which:
Figure 1 illustrates a perspective view of a syringe coupling according to an aspect of the present invention, shown adjacent but separate to a conventional syringe to which the syringe coupling is connectable;
Figure 2 illustrates the syringe coupling connected to the syringe as shown in Figure 1;
Figure 3 illustrates a sectioned perspective view of the arrangement shown in Figure 2;
Figure 4 illustrates a perspective view of the syringe coupling of the invention;
Figure 5 illustrates a side elevation of the syringe coupling;
Figure 6 illustrates a plan view from above of a microneedle based drug delivery device according to another aspect of the claimed invention;
Figure 7 illustrates a perspective view of one section of the drug delivery device shown in Figure 6;
Figure 8 illustrates a perspective view of a pair of seals and surrounding gaskets forming part of the syringe coupling shown in Figures 1 to 5, with the delivery device in an unassembled configuration;
Figure 9 illustrates the arrangement of Figure 8 with the delivery device in an assembled but undeployed configuration;
Figure 10 illustrates the pair of breaching elements being displaced through the pair of seals to rupture same such as to expose a pair of openings as the device is moved into the deployed configuration;
Figure 11 illustrates the pair of breaching elements having displaced the pair of seals such as to fully expose a pair of openings;
Figure 12 illustrates a perspective view of an alternative seal and surrounding gaskets and which may form part of the syringe coupling shown in Figures 1 to 5;
Figure 13 illustrates the arrangement of Figure 12 with a pair of breaching elements being advanced through the seal to expose a pair of openings;
Figure 14 illustrates a sectioned perspective view of the microneedle based delivery device shown in Figure 6 and to which the syringe as shown in Figures 2 to 5 is connected by way of the syringe coupling of the invention; and
Figure 15 illustrates a sectioned side elevation of the arrangement shown in Figure 14.

### Detailed description of the drawings

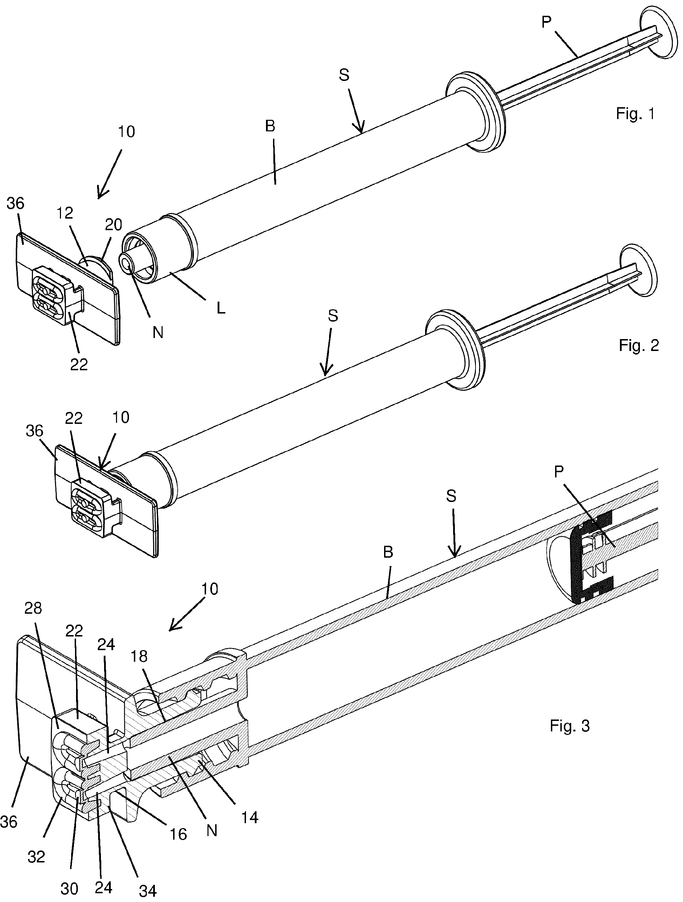

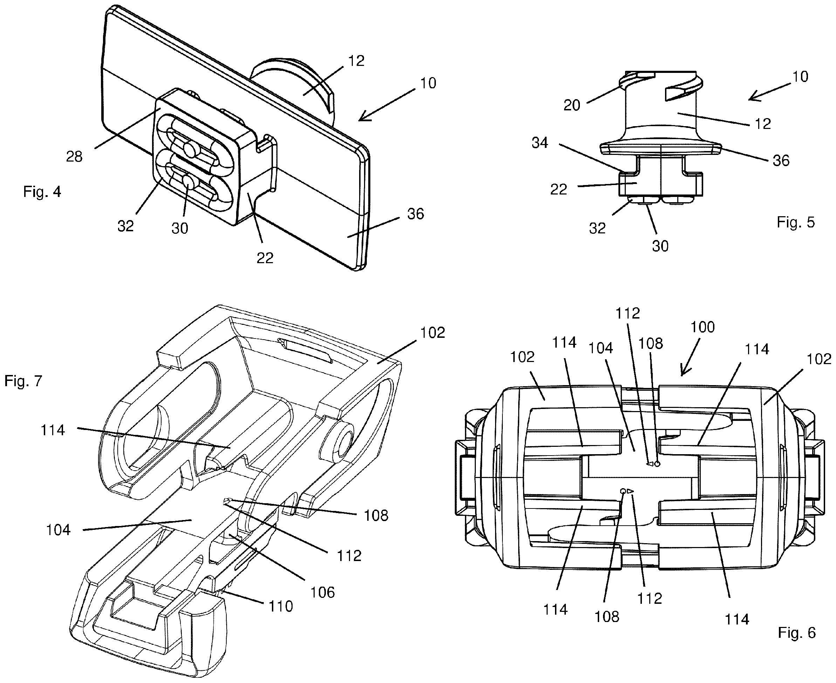

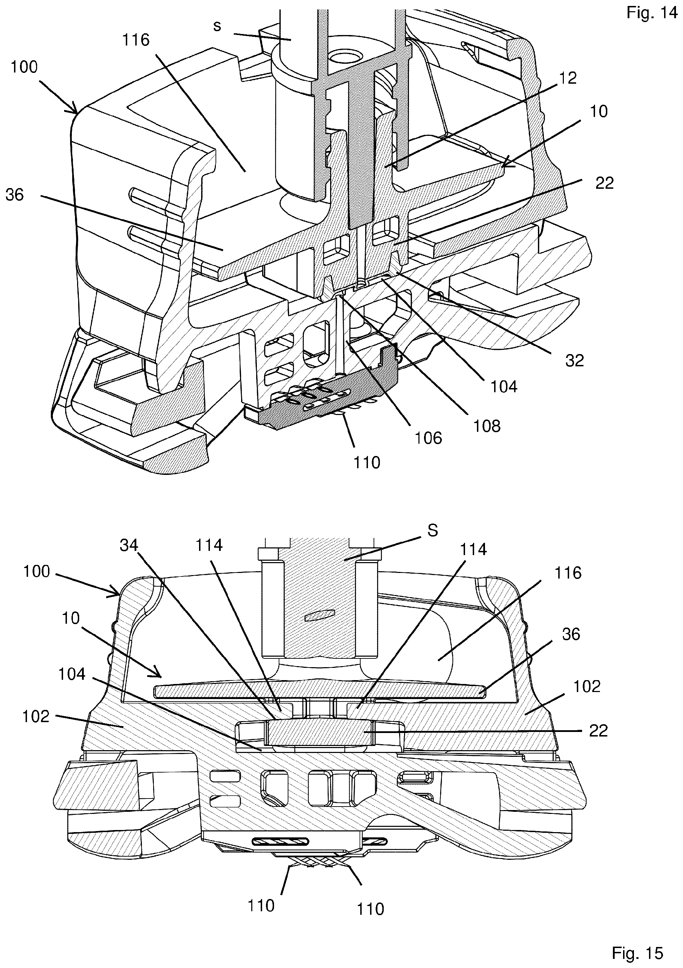

Referring now to Figures 1 to 5 of the accompanying drawings there is illustrated a syringe coupling, generally indicated as 10, for use in allowing a conventional syringe S, once filled with a medicament, to be hermetically sealed yet quickly and easily connected to a drug delivery device. The syringe coupling 10 is particularly adapted to allow such a conventional syringe S to be coupled with a microneedle based drug delivery device 100 as illustrated in Figures 6, 7, 14 and 15, being an example of the type of device described and shown in earlier International applications WO2018/069543, WO2022/238411 and WO2024/052432 in which a medicament may be injected through one or more microneedles which preferably also function to temporarily anchor the device to the target tissue. It will therefore be appreciated that reference to the drug delivery device 100 hereinafter should be construed as being a reference to the general design and function of the type of microneedle based devices disclosed in the above applications.

As described in detail hereinafter, the syringe coupling 10 acts to provide an interface between the conventional design of syringe S and the microneedle based drug delivery device 100, allowing the microneedle device 100 to deliver any suitable medicament once provided in said conventional design of syringe S, thereby taking advantage of well-established fill and finish manufacturing by which such conventional drug filled syringes are produced and filled. The syringe coupling 10 further provides a means of hermetically sealing the filled syringe S and a mechanism by which the seal may be automatically breached in response to deployment of the microneedle device 100 onto target tissue such as skin. In the following disclosure the syringe S comprises a barrel B and plunger P slidable therein to dispense the medicament contained within the syringe S, and preferably includes a conventional threaded Luer lock end fitting L, which the syringe coupling 10 is adapted for connection with as hereinafter described. It will however be appreciated that the syringe S may be of any other suitable configuration allowing a medicament to be dispensed therefrom, and any other suitable alternative to the Luer lock fitting L may be employed to allow the syringe S and syringe coupling 10 to be connected. Furthermore it should be understood that the syringe coupling 10 could be formed integrally with the syringe S.

The syringe coupling 10 comprises a cylindrical hub 12 having a first end 14 and an opposed second end 16, extending internally between which is a lumen 18. The hub 12 is preferably formed from a polymer but any suitable material or combination of materials may be employed. In the embodiment illustrated an exterior sidewall of the hub 12 comprises a thread 20 which cooperates with a corresponding thread in the Luer lock fitting L of the syringe S, allowing the syringe coupling 10 to be quickly and easily threaded into engagement with the syringe S, preferably by way of a suitable automated step during the fill and finish production of the syringe S. The Luer lock fitting L comprises a nozzle N which, once the syringe coupling 10 is fitted, is seated in fluid tight engagement within the lumen 18. The sidewall of both the lumen 18 and nozzle N may include a slight taper to facilitate a fluid tight interface therebetween as the nozzle N is drawn into the lumen 18. As noted above it will be understood that any other interface between the hub 12 and the syringe S may be employed.

The syringe coupling 10 further comprises a head 22 secured to or formed integrally with the hub 12 and positioned downstream thereof with respect to the direction of flow of a medicament from the barrel B in response to depression of the plunger P. As with the hub 12 the head 22 is preferably formed from a polymer. In the embodiment illustrated the head 22 is substantially rectangular in shape, as required to be captured within the microneedle based device 100 as hereinafter described. It will therefore be understood that the shape and/or dimensions of the head 22 may be varied as required to provide the necessary interface with any other design of microneedle based device. In use, and as described in detail hereinafter, the head 22 is utilised to capture and securely clamp the syringe coupling 10 within the microneedle based device 100,

The head 22 comprises a pair of internal passages 24 which open into the lumen 18 at an upstream end and terminate at openings 26 in a lower face 28 of the head 22 at a downstream end of the passages 24, such as to provide a fluid flow path from the hub 12, through the head 22, to the lower face 28. In the embodiment illustrated the lower face 28 extends transversely to a longitudinal axis of the syringe S but it will be appreciated that the geometry and orientation of the lower face 28 may be varied as required to sealingly mate with the microneedle based device 100 as hereinafter described Furthermore, while a pair of the passages 24 are provided it should be understood that a single passage or more than two passages may be utilised, in particular to reduce or increase the volume of medicament that can be dispensed through the syringe coupling 10 and/or to allow drug delivery through a greater or lesser number of microneedles on the microneedle based device 100.

The syringe coupling 10 further comprises a seal in the form of a pair of plugs 30, one secured about and/or partially within each of the openings 26, which serve to hermetically seal the passages 24 and thus establish a sterile enclosure for the medicament with the syringe S. The plugs 30 are formed from a deformable material, for example a thermoplastic elastomer and are secured in place by any suitable means, for example by way of an interference fit with the openings 26, adhesive, thermal bonding, overmoulding, or any other suitable means. Provided on the lower face 28, surrounding each opening 26 and the respective plug 30, are a pair of gaskets 32 each of which fully circumscribes the respective opening 26. The gaskets 32 are dimensioned to stand proud of the lower face 28 and are formed from a compressible material, for example a suitable thermoplastic elastomer, such as to be compressible against a sealing face of the microneedle based device 100 when the syringe coupling 10 is mated therewith, thus establishing a fluid tight seal with said sealing face. The gaskets 32 may be formed integrally with the head 22 and of the same material thereof, or may be formed from a distinct material and then suitably secured to the lower face 28 via adhesive, press fit, thermal bonding, overmoulding, or any other means. In the embodiment illustrated each gasket 32 is substantially oval in shape in order to surround the respective opening 26 and to accommodate the pair of gaskets 32 adjacent one another, but it will be understood that any other suitable shape and/or configuration may be employed.

The head 22 further comprises an upper face 34 which in use is engaged by the microneedle device 100 in order to allow the head 22, in particular the lower face 28 and gaskets 32, to be pressed against a cooperating sealing face of the microneedle device 100. In this way a fluid tight seal can be provided and maintained between the syringe S and the microneedle based device 100 in order to establish a closed fluid flow path for the medicament from the syringe S, through the microneedle based device 100, for delivery into the target tissue via one or more of the associated microneedles. This functionality is described in detail hereinafter.

One potential method of assembly is to capture the syringe coupling 10 within the microneedle based delivery device 100 and to then connect the syringe S to the syringe coupling. In order to isolate the syringe coupling 10 from rotational forces while the syringe S is being connected, which could result in misalignment of the syringe coupling 10 relative to the device 100, the syringe coupling 10 preferably comprises an torque plate 36 which, in the embodiment illustrated, extends in a direction transversely of a longitudinal axis of the syringe S and from a location intermediate the hub 12 and the head 22, although the exact position of the torque plate 36 may be varied while retaining the requisite functionality. The torque plate 36 provides a relatively rigid platform which may be constrained by the internal walls of the microneedle based device 100 and therefore utilised to prevent rotation of the syringe coupling 10 as the syringe S is threaded into engagement therewith. It will however be appreciated that the syringe S may first be connected to the syringe coupling 10 before the syringe coupling 10 is captured within the delivery device 100, and it is therefore possible that the torque plate 34 could be omitted from the syringe coupling 10.

Referring now to Figures 6, 7, 14 and 15 the microneedle based drug delivery device 100 is shown which, in the embodiment illustrated, comprises a pair of identical halves or sections 102 which are connected together. One of the sections 102 is shown in isolation in Figure 7 while the complete device 100, consisting of a pair of the interlocked sections 102 is shown in Figure 6. WO2022/238411 provides a more detailed explanation of the general configuration and operation of the microneedle based device 100, the requisite details of which are incorporated herein in their entirety. In summary the device 100, once assembled with the syringe S and syringe coupling 10 captured therein, is displaceable between an undeployed state in which the sections 102 are partially separated from one another as shown in Figure 6, and a deployed state in which the sections 102 are pressed fully into engagement with one another. The device 100 is displaced from the undeployed to the deployed state while in contact with the target tissue, the relative displacement between the sections 102 drawing opposed sets of microneedles 110, projecting from an tissue contacting surface on an underside of the device 100, into the tissue in order to anchor the device 100 in place. Medicament can then be delivered through the microneedles 110 into the target tissue as will be described.

When connecting the two sections 102 together to initially assemble the device 100 into the undeployed configuration shown in Figure 6, the syringe coupling 10, with or without the syringe S secured thereto, is positioned between the pair of sections 102 which are then advanced into engagement with one another as shown in Figure 6. The pair of plugs 30 are in place at this stage, ensuring the medicament remains hermetically sealed within the syringe S if already connected to the syringe coupling 10. The action of sliding the sections 102 into initial contact with one another captures the syringe coupling 10 between the sections 102, in particular engaging and gripping the head 22 between the sections 102, thereby securing the syringe coupling 10 to the microneedle based device 100. In the embodiment illustrated each section 102 comprises a sealing face 104 which defines an upper end of a manifold 106 which provides a fluid flow path between the sealing face 104 and the respective array of microneedles 110. Each sealing face 104 includes an inlet 108 therein which defines an upper end of the fluid flow path which extends downwardly through the manifold 106 and into one or more of the microneedles 110. When the device 100 is assembled the pair of sealing faces 104 abut to form a continuous surface against which the lower face 28 is located, and slide relative to one another as the device 100 is displaced into the deployed state. In the undeployed state the pair of inlets 108 are offset, but when displaced into the deployed state are aligned with one another. In use, as described hereinafter, each inlet 108 is brought into register with one of the openings 26 as the device 100 is displaced into the deployed state to allow the medicament to flow from the syringe coupling 10 though the microneedle based device 100 to the microneedles 110 and ultimately into the target tissue. Located on and upstanding of the sealing face 104 adjacent each inlet 108 is a breaching element 112 which may be pointed, and which is adapted to move or deformed the respective plug 30, as the device 100 is displaced into the deployed state, in order to expose the opening 26 and connect the fluid flow paths of the syringe coupling 10 and the microneedle based device 100.

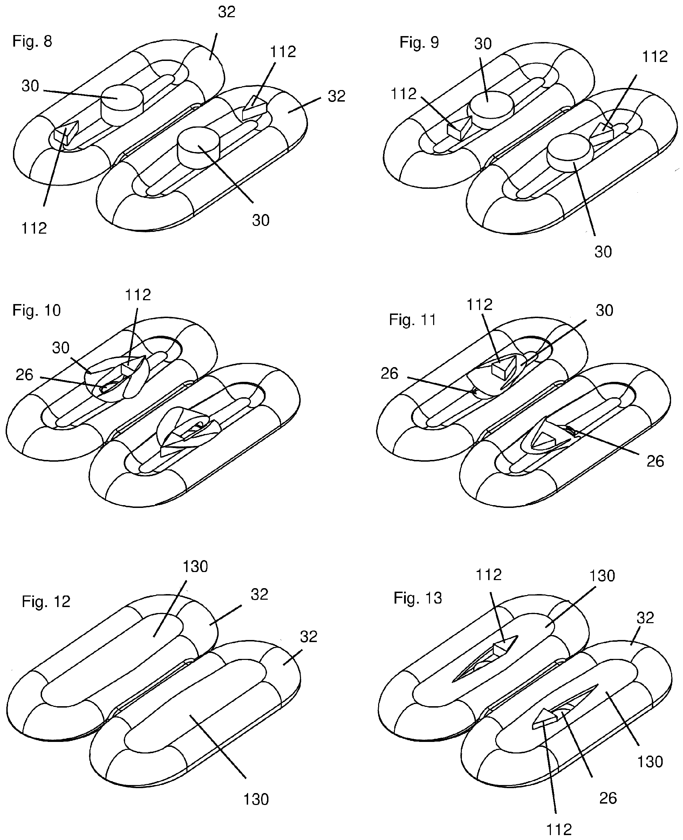

Thus when assembling the device 100, the lower face 28 is placed in face to face contact with the pair of sealing faces 104 such that the gaskets 32 lightly contact the sealing face 104, each gasket 32 surrounding one of the inlets 108 and associated breaching element 112. The relative positions of the plugs 30, gaskets 32 and breaching elements 112 in the un-assembled configuration is shown in Figure 8 which provides clearance between the gaskets 32 and the breaching element 112, which is important to ensure that the gaskets 32 are not compromised during assembly. Figure 9 shows the relative positions with the delivery device 100 in the undeployed configuration in which the opposed outer edges of the upper face 34 of the head 22 are each captured beneath a bearing plate 114 provided on each section 102, spaced a small distance upwardly from the sealing face 104 in order to accommodate the edge of the head 22 therebetween. The torque plate 36 is vertically spaced or separated from the head 22, in particular the upper face 34, in order to accommodate the opposed bearing plates 114. The edges of the head 22 are initially captured between the bearing plates 114 and the combined sealing faces 104 when the device 100 is initially assembled into the undeployed state as illustrated in Figure 7. The bearing plates 114 have a gradual lengthwise taper on the side facing the sealing faces 104 and thus in contact with the upper face 34 which includes a cooperating taper as hereinbefore described. Thus as the sections 102 are advanced towards one another as the device 100 is displaced into the deployed state, the tapered underside of the bearing plates 114 bear down on the upper face 34 of the head 22, urging the gaskets 32 against the sealing faces 104, compressing the gaskets 32 and establishing a fluid tight seal between the gaskets 32 and the sealing faces 104. At this stage, if the syringe S is already connected to the syringe coupling 10, the device 100 is configured to allow the medicament to be dispensed from the connected syringe S, through the device 100 via the syringe coupling 10, into the target tissue via the microneedles 104. However if the syringe 10 has not yet been connected to the syringe coupling 10 it is now threaded into engagement therewith. As this action will exert a torque on the syringe coupling 10, which could result in misalignment of the openings 26 and inlets 108, it is important that the syringe coupling 10 is rotationally immobilised relative to the microneedle based device 100. The torque plate 36 provides this functionality, being shaped and dimensioned to be closely captured between opposed lateral walls 116 of the delivery device 100, thereby preventing the torque plate 36, and by extension the syringe coupling 10, from undergoing rotational displacement relative to the delivery device 100 as the syringe S is threaded onto the syringe coupling 10. The filled syringe S can therefore be connected to the syringe coupling 10, and the plugs 30 will ensure that the medicament remains hermetically sealed within the syringe S until the delivery device 100 is ready for use. However as noted above the syringe coupling 10 is preferably already connected to the syringe S before the syringe coupling 10 and delivery device 100 are connected together.

However as noted hereinbefore, in order to allow the medicament to be dispensed from the syringe S, the pair of openings 26 must be exposed, which is achieved by displacing the pair of breaching elements 112 into the plugs 30 to split, tear, displace or otherwise reconfigure the plugs 30 such as to expose the openings 26. Referring to Figures 9 to 11 the sequence of steps by which this is achieved is illustrated. Figure 9 illustrates the relative positions of the plugs 30, gaskets 32 and breaching elements 112 when the device 100 is in the undeployed state. It can be see that the plugs 30 and gaskets 32 are slightly compressed as the lower face 28 has not yet been urged tightly against the sealing faces 104. The breaching elements 112 are located remote from the respective plug 30, corresponding to the configuration of the device 100 as shown in Figure 6.

Figure 10 illustrates the relative positions as the device 100 is being moved from the undeployed to the deployed state. At this stage the bearing plate 114 is pressing downwardly on the upper face 34 forcing the lower face 28 of the head 22 against the sealing faces 104 and thereby compressing the plugs 30 and gaskets 32 to form a fluid tight seal around the plugs 30. At the same time the relative displacement of the sections 102 has resulted in the pair of breaching elements 112 being moved towards the plugs 30 along two perpendicular directions, namely having both a vertical and horizontal component of motion.

Figure 10 illustrates the arrangement with the device 100 in the deployed state, with the pair of breaching elements 112 having deformed the plugs 30 such as to expose the openings 26.

Figure 11 illustrates the arrangement with the device 100 in the fully deployed state, with the pair of breaching elements 112 having completed the requisite deformation of the plugs 30, leaving the openings 26 exposed to allow medicament to flow from the openings 26 and through the inlets 108 which will now be aligned with the openings 26. It will therefore be appreciated that the hermetic sealing of the syringe S remains intact until the device 100 is fully deployed on the target tissue. In this state the device 100 is firmly anchored to the target tissue by way of the embedded microneedles 110, and the plunger P can therefore be depressed to deliver the medicament from the syringe S into the target tissue through the microneedles 110.

Figures 12 and 13 shown an alternative form of seal in the form of rupturable membranes 130 occluding the openings 26. The breaching elements 112 are brought into register with membranes 130 as the gaskets 32 are pressed against the sealing faces 104. The linear displacement of the breaching elements 112 along two perpendicular directions as hereinbefore described then ruptures or tears the membranes 130, exposing the openings 26 and allowing fluid flow therefrom.

It will be appreciated from the above that the syringe coupling 10 of the invention provides a mechanism by which a conventional syringe S can be utilised with contemporary microneedle based drug delivery devices, allowing a medicament to be maintained under sterile conditions within the syringe S, with the seal only being broken as the microneedle based delivery device is being deployed onto the target tissue. Thus the efficiencies of well-established fill and finish manufacturing can be retained while availing of the benefits arising from the use of microneedle based drug delivery, all which ensuring the integrity of the medicament.

The invention is not limited to the embodiment described herein but can be amended or modified without departing from the scope of the present invention.

## Claims

1. A syringe coupling comprising a hub having a first end sealingly connectable to a barrel of a syringe and an opposed second end between which is defined a fluid flow lumen; a head provided at the second end of the hub and comprising a lower face defining at least one opening therein, the head further comprising at least one passage communicating between the lumen and the at least one opening; and a seal located about the at least one opening and displaceable from a first state occluding the opening to a second state exposing the opening.

2. A syringe coupling according to claim 1 in which the seal comprises a deformable material.

3. A syringe coupling according to claim 1 or 2 in which the seal comprises a plug at least partially disposed in the at least one opening.

4. A syringe coupling according to claim 1 or 2 in which the seal comprises a rupturable membrane extending over at least the at least one opening.

5. A syringe coupling according to any preceding claim further comprising at least one gasket on the lower face and surrounding the at least one opening.

6. A syringe coupling according to claim 4 in which the at least one gasket comprises a compressible material.

7. A syringe coupling according to any preceding claim comprising a torque plate operable to prevent relative rotation of the syringe coupling when captured with a microneedle based delivery device.

8. A syringe coupling according to claim 6 in which the torque plate extends transversely to a longitudinal axis of the hub.

9. A syringe coupling according to any of claims 6 to 8 in which the torque plate is rectangular.

10. A syringe according to claim 6 or 7 in which the torque plate is located intermediate the hub and the head.

11. A syringe coupling according to any preceding claim in which the hub comprises a thread for engaging with a corresponding thread on the syringe.

12. A syringe coupling according to any preceding claim in which the lower face defines a pair of openings therein, the head further comprising a corresponding pair of passages communicating between the lumen and the pair of openings.

13. A microneedle based drug delivery device comprising a body having a first section and a second section displaceable relative to one another; at least one first microneedle provided on the first section and at least one second microneedle provided on the second section, at least one of the first and at least one second microneedles being hollow such as to facilitate drug delivery therethrough, the first and second sections being displaceable relative to one another in order to transition the first and second microneedles between a disengaged state and an engaged state; a delivery manifold in fluid communication with the at least one hollow microneedle and defining a seat against which the lower face of the head of the syringe coupling of claims 1 to 12 is sealably engagable; and at least one breaching element displaceable in response to relative displacement of the first and second sections such as to effect displacement of the seal from the first state into the second state.

14. The microneedle based delivery system of claim 13 in which the syringe coupling is captured between the first and second sections at least when the microneedles are in the engaged state.

15. The microneedle based delivery system of claim 13 or 14 in which the lower face is urged against the seat when the microneedles are in the engaged state such as to establish a fluid tight seal between the lower face and the seat.
